# FASCICULE DE BREVET EUROPEEN

(11) **EP 1 530 979 B1**
(45) Date de publication et mention de la délivrance du brevet: **14.01.2009**
(21) Numéro de dépôt: 04292668.3
(22) Date de dépôt: 10.11.2004
(51) Int. Cl.: A61M 5/315

(54) **Ensemble de sécurité destiné à équiper une seringue et ensemble de seringue**
Sicherheitseinrichtung für eine Spritzvorrichtung
Security assembly for a syringe device

(30) Priorité: 14.11.2003 FR 0313366; 04.12.2003 FR 0314268
(43) Date de publication de la demande: 18.05.2005
(73) Titulaire: Rexam Pharma La Verpillière, 38290 La Verpillière Cedex (FR)
(72) Inventeur: Rimlinger, Thierry, 38080 L'Isle D'Abeau (FR); Belle, Guillaume, 38470 Vinay (FR); Dugand, Pascal, 38780 Estrablin (FR)
(74) Mandataire: de la Bigne, Guillaume Michel Marie

(56) Documents cités:
- EP-A- 0 242 956
- WO-A-00/69492
- FR-A- 2 245 382
- FR-A- 2 799 375
- FR-A- 2 830 199

## Description

La présente invention concerne un ensemble de sécurité destiné à équiper une seringue et un ensemble de seringue.

Certains produits médicaux sont conditionnés directement dans un corps de seringue standard (seringue pré-remplie), généralement en verre, obturé par un piston auquel est relié un poussoir d'actionnement du piston.

Le corps d'une seringue est muni généralement d'une extrémité de raccordement à une aiguille d'injection, dite extrémité d'injection, et d'une extrémité d'introduction du piston. On peut donc accéder au produit contenu dans la seringue par chacune de ces extrémités par exemple soit en retirant le piston du corps de la seringue soit en refoulant ou aspirant le produit à travers l'aiguille. On peut également accéder au produit contenu dans la seringue au moyen d'une aiguille d'injection que l'on enfonce à travers le piston, généralement en élastomère, après avoir enlevé le poussoir.

On connaît déjà dans l'état de la technique, notamment d'après FR 2 799 375 A1 (FR 99 12500), un ensemble de sécurité destiné à équiper une seringue comportant un corps muni d'une extrémité de raccordement à une aiguille d'injection, dite extrémité d'injection, et d'une extrémité d'introduction d'un piston, l'ensemble de sécurité étant du type comprenant :
- un organe destiné à être rapporté sur le corps de la seringue de façon solidaire axialement de ce corps, et
- un capuchon de sécurité, destiné à recouvrir l'extrémité d'injection du corps, muni d'une partie proximale d'accrochage sur l'organe rapporté et d'une partie distale de dégagement de l'extrémité d'injection reliée à la partie d'accrochage par des moyens frangibles.

Après accrochage sur le corps de la seringue de la partie proximale du capuchon de sécurité, l'extrémité d'injection du corps de la seringue ne peut être dégagée qu'après rupture des moyens frangibles. Ainsi, un accès frauduleux au produit contenu dans la seringue par l'extrémité d'injection du corps de cette seringue est révélé par la rupture des moyens frangibles.

On connaît par ailleurs dans l'état de la technique, notamment d'après DE 29 45 869 A1, FR 2 245 382, WO 00/69492, FR 2 830 199 ou encore EP 0 242 956, un organe de retenue du piston à l'intérieur du corps de la seringue permettant notamment d'empêcher le retrait intempestif ou frauduleux de ce piston.

L'invention a notamment pour but de déceler et/ou d'éviter un accès frauduleux au produit contenu dans la seringue, en particulier lorsque cette seringue est proposée à la vente, ceci à l'aide de moyens simples et efficaces.

A cet effet, l'invention a pour objet un ensemble de sécurité destiné à équiper une seringue, du type pré-cité, caractérisé en ce que l'organe rapporté forme un organe destiné à retenir le piston à l'intérieur du corps.

Ainsi, l'ensemble de sécurité selon l'invention permet, à la fois, de n'autoriser l'accès à l'extrémité d'injection du corps de la seringue (munie ou non d'une aiguille) qu'après rupture des moyens frangibles (si bien qu'un accès frauduleux au produit contenu dans la seringue par l'extrémité d'injection du corps de cette seringue est révélé par cette rupture des moyens frangibles) et d'empêcher le retrait intempestif ou frauduleux du piston ou/et du poussoir de la seringue.

Suivant d'autres caractéristiques optionnelles de cet ensemble de sécurité :
- la partie d'accrochage du capuchon de sécurité forme des moyens de retenue radiale de l'organe rapporté sur le corps de la seringue ;
- l'organe rapporté comprend au moins un secteur de jupe annulaire, dit secteur de centrage, destiné à coopérer avec une surface complémentaire délimitant le corps de la seringue pour positionner radialement l'organe rapporté par rapport au corps de la seringue ;
- le capuchon de sécurité est accroché sur l'organe rapporté à l'aide de moyens d'encliquetage complémentaires portés par la partie d'accrochage du capuchon de sécurité et l'organe rapporté, ces moyens d'encliquetage complémentaires étant emboîtables l'un dans l'autre de façon irréversible ;
- les moyens d'encliquetage complémentaires comprennent des épaulements complémentaires de verrouillage ménagés, respectivement, sur une extrémité sensiblement annulaire de la partie d'accrochage du capuchon de sécurité et sur au moins un secteur de jupe annulaire solidaire de l'organe rapporté, dit secteur d'encliquetage ;
- le secteur de centrage est radialement interne par rapport au secteur d'encliquetage, l'extrémité sensiblement annulaire de la partie d'accrochage du capuchon de sécurité étant intercalée radialement entre ces secteurs de centrage et d'encliquetage ;
- l'épaulement de verrouillage ménagé sur le secteur d'encliquetage est adjacent à des fentes circonférentielles ménagées dans ce secteur d'encliquetage ;
- l'ensemble de sécurité étant destiné à équiper une seringue comportant un corps muni d'une extrémité d'introduction d'un piston délimitée par une collerette, l'organe rapporté comprend des flasques proximal et distal destinés à pincer axialement la collerette pour solidariser axialement l'organe rapporté au corps de la seringue, le flasque proximal formant un collet destiné à retenir le piston à l'intérieur du corps ;
- le flasque distal porte le secteur de centrage ;
- le flasque distal porte les moyens d'encliquetage destinés à coopérer avec les moyens d'encliquetage complémentaires portés par la partie d'accrochage du capuchon de sécurité ;
- le flasque distal comporte une ouverture destinée à l'emboîtement autour du corps de la seringue, cette ouverture d'emboîtement débouchant dans une direction sensiblement parallèle aux flasques, le contour du flasque distal délimitant cette ouverture d'emboîtement ayant de préférence une forme en "U" ;
- l'ensemble de sécurité étant destiné à équiper une seringue comportant un corps muni d'un piston, l'ensemble de sécurité comprend une tige formant un poussoir destinée à être relié au piston et le flasque proximal comporte une ouverture destinée au débattement axial du poussoir, cette ouverture de débattement débouchant de préférence dans une direction sensiblement parallèle aux flasques, le contour du flasque proximal délimitant l'ouverture de débattement formant le collet de retenue du piston et ayant de préférence une forme en "U" ;
- le poussoir comporte une extrémité distale interne au corps munie d'un disque formant une protection anti-perçage du piston et/ou une butée destinée à coopérer avec le flasque proximal formant collet pour retenir le piston et l'extrémité distale du poussoir à l'intérieur du corps ;
- l'organe rapporté comprend des moyens d'immobilisation en rotation destinés à coopérer avec des moyens complémentaires de la collerette du corps de la seringue, ces moyens d'immobilisation en rotation étant munis de préférence de méplats ménagés dans l'organe rapporté de façon à s'étendre axialement entre les deux flasques ;
- l'organe rapporté est muni de moyens d'appui de doigts d'un utilisateur comprenant de préférence deux ailes de préhension sensiblement diamétralement opposées ;
- le capuchon de sécurité a une forme générale de révolution, les moyens frangibles comprenant une fente annulaire, séparant les deux parties du capuchon de sécurité, cette fente étant interrompue par des ponts frangibles reliant les deux parties du capuchon de sécurité ;
- les parties de dégagement et d'accrochage du capuchon de sécurité sont munies de moyens d'accrochage complémentaires susceptibles de coopérer entre eux après rupture des moyens frangibles ;
- les moyens d'accrochage complémentaires comprennent des moyens d'encliquetage complémentaires emboîtables munis d'épaulements complémentaires de verrouillage et de butées complémentaires de fin de course d'emboîtement ménagés sur les parties, respectivement, de dégagement et d'accrochage du capuchon de sécurité ;
- les butées complémentaires de fin de course sont délimitées, respectivement, par une saillie externe, par exemple annulaire, ménagée sur l'une des parties du capuchon de sécurité et un bord de l'autre des parties du capuchon de sécurité délimitant la fente annulaire ;
- le capuchon de sécurité est fabriqué dans un matériau transparent afin notamment de permettre un accès visuel à une étiquette portée par la seringue ;
- la partie de dégagement du capuchon de sécurité est destinée à former un capuchon de protection de l'aiguille d'injection ;
- la partie de dégagement du capuchon de sécurité est destinée à être reliée à un capuchon de protection de l'aiguille d'injection.

L'invention a également pour objet un ensemble de seringue du type comprenant une seringue comportant un corps muni d'une extrémité de raccordement à une aiguille d'injection, dite extrémité d'injection, et d'une extrémité d'introduction d'un piston, caractérisé en ce qu'il comprend de plus un ensemble de sécurité tel que défini précédemment.

L'invention sera mieux comprise à la lecture de la description qui va suivre, donnée uniquement à titre d'exemple et faite en se référant aux dessins annexés dans lesquels :
- la figure 1 est une vue en perspective d'un ensemble de seringue selon un premier mode de réalisation de l'invention ;
- la figure 2 est une vue en coupe axiale de l'ensemble de seringue représenté sur la figure 1 ;
- les figures 3 à 5 sont des vues en perspective, partiellement éclatées, d'une partie au moins de l'ensemble de seringue représenté sur la figure 1 dans différentes configurations de montage ou d'utilisation ;
- les figures 6 et 7 sont des vues en perspective, suivant deux points de vue différents, d'un organe destiné à être rapporté sur le corps de la seringue de l'ensemble de seringue représenté sur la figure 1 ;
- la figure 8 est une vue similaire à la figure 1 d'un ensemble de seringue selon un second mode de réalisation de l'invention ;
- les figures 9 et 10 sont des vues partielles en coupe axiale de l'ensemble de seringue représenté sur la figure 8 montrant le capuchon de sécurité avant et après une première utilisation de la seringue.

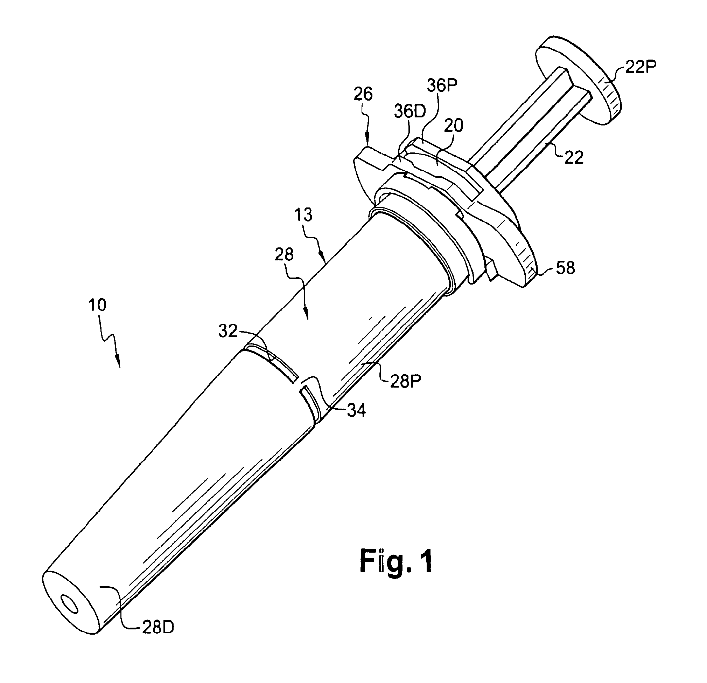

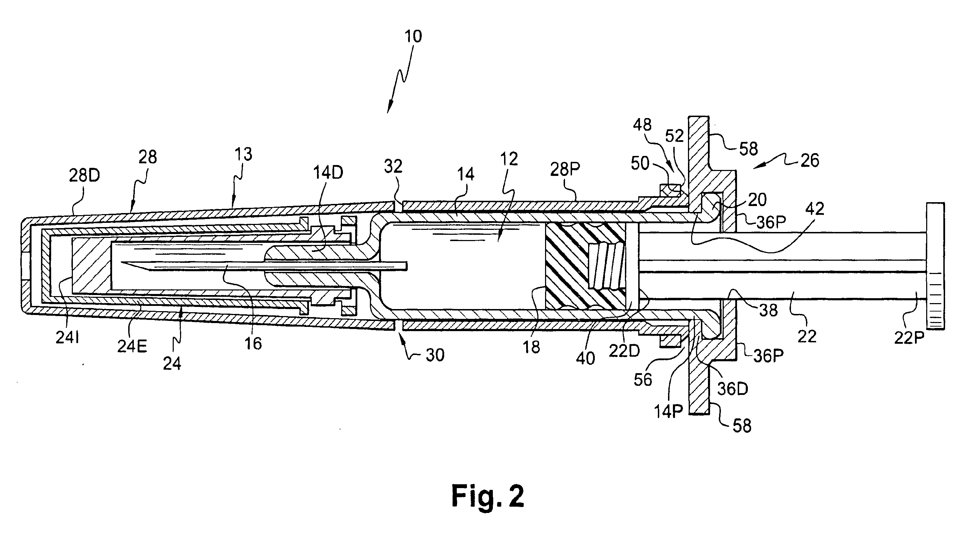

On a représenté sur les figures 1 et 2 un ensemble de seringue selon un premier mode de réalisation de l'invention, désigné par la référence générale 10.

L'ensemble 10 de seringue comprend une seringue 12, destinée à l'injection d'un liquide, notamment de type médical, équipée d'un ensemble de sécurité 13 selon l'invention.

Dans ce qui suit, on qualifiera de proximal un élément de l'ensemble de seringue ou de sécurité proche axialement de la main d'un utilisateur et de distal un élément de cet ensemble de seringue ou de sécurité éloigné de la main de l'utilisateur.

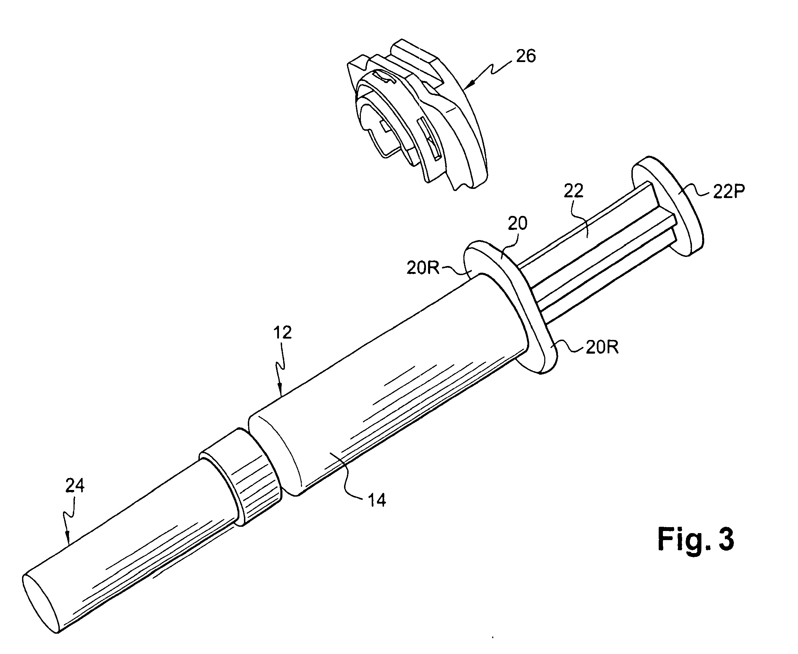

De façon classique, la seringue 12, représentée notamment sur les figures 2 et 3, comporte un corps tubulaire 14, par exemple en verre. Ce corps 14 est muni d'une extrémité distale 14D de raccordement à une aiguille d'injection 16. Cette extrémité 14D sera appelée par la suite extrémité d'injection. Le corps 14 est également muni d'une extrémité proximale 14P ouverte autorisant l'introduction d'un piston 18 dans le corps 14. Ce piston 18, de type classique, est par exemple en élastomère.

On notera que l'extrémité d'introduction 14P est délimitée par une collerette 20. Dans l'exemple décrit, la collerette 20 est délimitée par un contour circulaire interrompu par deux parties sensiblement rectilignes 20R, diamétralement opposées, visibles notamment sur la figure 3. En variante, la collerette peut être délimitée par un contour circulaire ininterrompu.

L'ensemble de sécurité 13 comprend un poussoir de sécurité 22 muni d'une extrémité distale 22D, interne au corps 14, reliée, par exemple par vissage, au piston 18, et d'une extrémité proximale 22P, externe au corps 14, formant extrémité de manoeuvre du poussoir 22.

L'extrémité d'injection 14D du corps et l'aiguille 16 sont recouvertes par un capuchon de protection classique 24 muni, dans l'exemple décrit, d'une enveloppe externe 24E et d'une enveloppe interne 241 par exemple en élastomère.

L'ensemble de sécurité 13 comprend également un organe 26 rapporté sur le corps 14 et un capuchon de sécurité 28 recouvrant, en les entourant, le capuchon de protection 24, l'aiguille 16 et l'extrémité d'injection 14D du corps de la seringue.

Le capuchon de sécurité 28 est muni d'une partie proximale 28P destinée à être accrochée sur le corps 14 de la seringue, par l'intermédiaire de l'organe rapporté 26, et d'une partie distale 28D de dégagement de l'extrémité d'injection 14D du corps de la seringue. La partie d'accrochage 28P est reliée à la partie de dégagement 28D à l'aide de moyens frangibles 30.

On notera que la seringue 12 et le capuchon de sécurité 28 ont des formes générales de révolution.

Les moyens frangibles 30 du capuchon de sécurité 28 comprennent par exemple, une fente annulaire 32, séparant les deux parties 28D, 28P de ce capuchon de sécurité. La fente 32 est interrompue par des ponts frangibles 34 reliant les deux parties 28D et 28P du capuchon de sécurité.

De préférence, le capuchon de sécurité 28 est fabriqué dans un matériau transparent, par exemple en plastique, afin notamment de permettre un accès visuel à une étiquette portée par la seringue 12, par exemple par le capuchon de protection 24.

Le positionnement axial des moyens frangibles 30 sur le capuchon de sécurité 28 peut varier selon les cas. De préférence, lorsque la seringue 12 porte une étiquette destinée à être décollée ou pelée, les moyens frangibles 30 sont positionnés sur le capuchon de sécurité 28 de façon à permettre, après rupture de ces moyens frangibles, d'accéder à l'étiquette pour la décoller ou la peler.

Suivant une première variante, le capuchon de protection 24 peut être supprimé, la partie de dégagement 28D du capuchon de sécurité 28 formant alors un capuchon de protection de l'aiguille d'injection 16.

Suivant une seconde variante, la partie de dégagement 28D du capuchon de sécurité peut être reliée au capuchon de protection 24 de façon que la séparation de la partie de dégagement 28D d'avec la partie d'accrochage 28P du capuchon de sécurité entraîne la séparation du capuchon de protection 24 d'avec l'extrémité d'injection 14D du corps de la seringue.

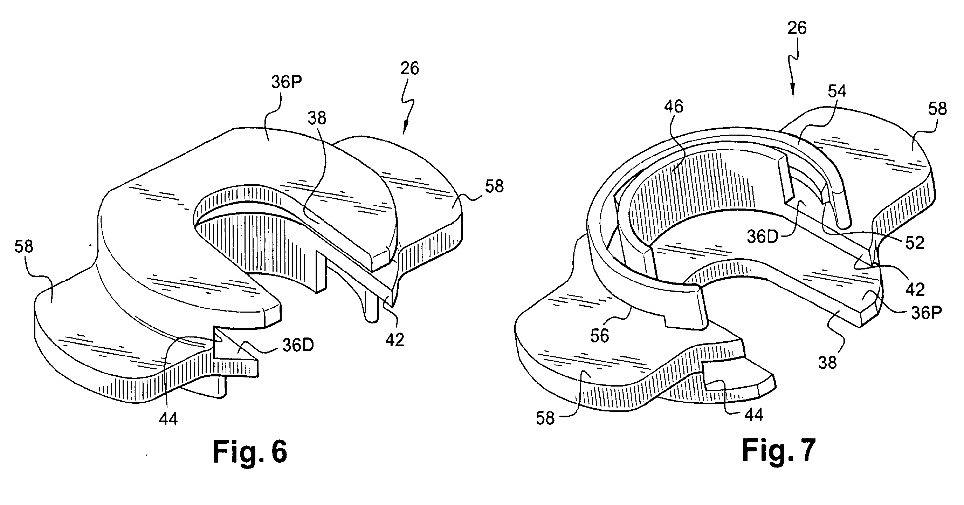

En se référant notamment, d'une part, aux figures 1 et 2, et d'autre part, aux figures 6 et 7 dans lesquelles l'organe rapporté 26 est représenté plus en détail, on voit que cet organe rapporté 26 comprend des flasques proximal 36P et distal 36D destinés à pincer axialement la collerette 20 pour solidariser axialement l'organe rapporté 26 au corps 14 de la seringue.

L'organe rapporté 26 forme un organe destiné à retenir le piston 18 et l'extrémité distale 22D du poussoir à l'intérieur du corps 14 de la seringue. En effet, le flasque proximal 36P forme un collet de retenue du piston 18 à l'intérieur du corps 14.

On notera plus particulièrement que le flasque proximal 36P comporte une ouverture 38 destinée au débattement axial du poussoir 22. Le contour du flasque proximal 36P délimitant cette ouverture de débattement 38 forme le collet de retenue du piston 18. De préférence, l'ouverture de débattement 18 débouche dans une direction sensiblement parallèle aux flasques 36P, 36D, c'est-à-dire dans une direction sensiblement perpendiculaire à l'axe de la seringue 12. Le contour du flasque proximal 36P délimitant cette ouverture 38 a de préférence une forme en "U" de manière à présenter des bords sensiblement rectilignes et parallèles écartés entre eux d'une distance adaptée pour autoriser un déplacement transversal du flasque proximal 36P relativement au poussoir 22.

On notera que l'extrémité interne 22D du poussoir est munie d'un disque 40 formant une butée de retenue du piston 18 destinée à coopérer avec le flasque proximal 36P formant collet. Dans l'exemple illustré, le disque 40 est adjacent axialement au piston 18. Par ailleurs, le disque 40 forme une protection anti-perçage du piston 18.

Le flasque distal 36D comporte une ouverture 42 destinée à l'emboîtement de ce flasque distal 36D autour du corps 14 de la seringue. Cette ouverture d'emboîtement 42 débouche dans une direction sensiblement parallèle aux flasques 36D, 36P, c'est-à-dire dans une direction sensiblement perpendiculaire à l'axe de la seringue 12. Le contour du flasque distal 36D délimitant l'ouverture d'emboîtement 42 a de préférence une forme en "U" de manière à présenter des bords sensiblement rectilignes et parallèles écartés entre eux d'une distance adaptée pour autoriser un emboîtement transversal du flasque distal 36D sur le corps 14 de la seringue.

De préférence, l'organe rapporté 26 comprend une paire de méplats 44, diamétralement opposés, ménagés dans cet organe rapporté 26 de façon à s'étendre axialement entre les deux flasques 36P, 36D. Ces méplats 44 peuvent former des moyens d'immobilisation en rotation de l'organe rapporté 26 par rapport au corps 14 de la seringue destinés à coopérer avec les bords rectilignes 20R de la collerette formant des moyens d'immobilisation en rotation complémentaires.

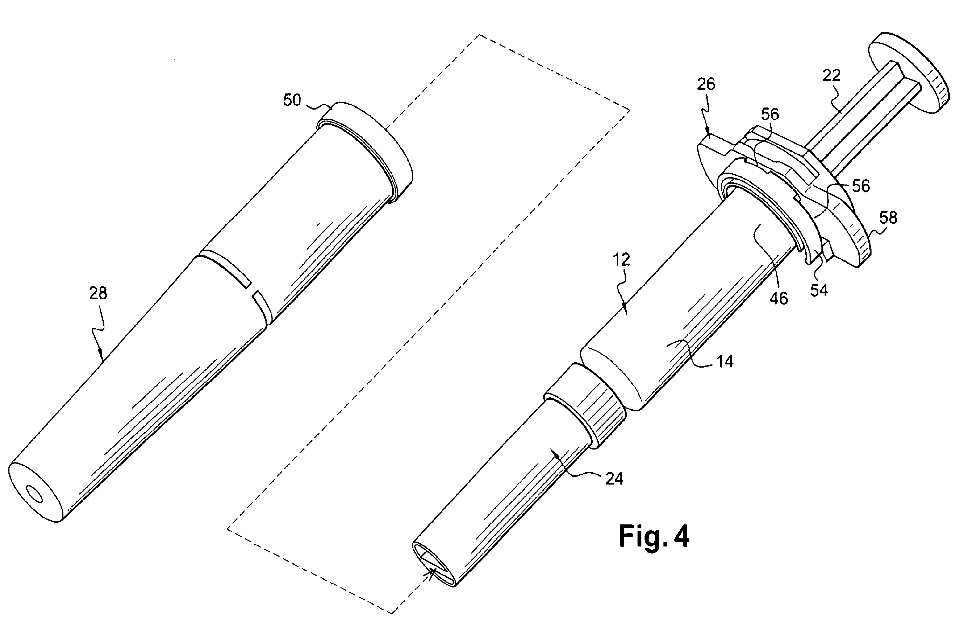

En se référant notamment aux figures 4, 6 et 7, on voit que le flasque distal 36D comprend au moins un secteur 46 de jupe annulaire, dit secteur de centrage, destiné à coopérer avec une surface complémentaire délimitant le corps 14 de la seringue pour positionner radialement l'organe rapporté 26 par rapport à ce corps 14. Dans l'exemple illustré, le secteur de centrage 46, venu de matière avec le flasque distal 36D, s'étend sensiblement sur 180° et coopère avec la surface externe du corps 14 de la seringue.

Le capuchon de sécurité 28 est destiné à être accroché sur l'organe rapporté 26. Après accrochage sur cet organe rapporté 26, le capuchon de sécurité 28 s'étend autour du corps 14 de la seringue sensiblement coaxialement à ce corps 14. Ainsi, le capuchon de sécurité 28, plus particulièrement la partie d'accrochage 28P de ce dernier, forme des moyens de retenue radiale de l'organe rapporté 26 sur le corps 14 de la seringue.

De préférence, le capuchon de sécurité 28 est accroché sur l'organe rapporté 26 à l'aide de moyens d'encliquetage complémentaires 48 portés par la partie d'accrochage 28P du capuchon de sécurité et l'organe rapporté 26. Ces moyens d'encliquetage complémentaires 48 sont emboîtables l'un dans l'autre de façon irréversible, c'est à dire qu'un déboîtement éventuel de ces moyens d'encliquetage 48 laisse une trace visible.

Dans l'exemple représenté sur les figures, les moyens d'encliquetage complémentaires 48 comprennent des épaulements complémentaires de verrouillage 50, 52, ménagés, respectivement, sur une extrémité sensiblement annulaire de la partie d'accrochage 28P du capuchon de sécurité (voir notamment figures 2 et 4) et sur au moins un secteur 54 de jupe annulaire venu de matière avec le flasque distal 36D de l'organe rapporté (voir notamment figures 2 et 7). Par la suite, le secteur 54 sera appelé secteur d'encliquetage.

Dans l'exemple illustré, le secteur de centrage 46 s'étend sur plus de 180°.

On notera, en se référant notamment aux figures 1 et 7, que l'épaulement de verrouillage 52, ménagé sur le secteur d'encliquetage 54, est adjacent axialement à des fentes circonférentielles 56 ménagées dans ce secteur d'encliquetage 54. Ces fentes 56 favorisent l'encliquetage des moyens complémentaires 48.

On notera également, en se référant notamment les figures 4 et 7, que le secteur de centrage 46 est radialement interne par rapport au secteur d'encliquetage 54. Après encliquetage des moyens complémentaires 48, l'extrémité sensiblement annulaire de la partie d'accrochage 28P du capuchon de sécurité est intercalée radialement entre les secteurs de centrage 46 et d'encliquetage 54.

De préférence, l'organe rapporté 26 est muni de moyens d'appui de doigts d'un utilisateur comprenant, par exemple, deux ailes de préhension radiales 58 sensiblement diamétralement opposées.

On notera que l'ensemble de sécurité 13 peut être commercialisé indépendamment de la seringue 12 sous la forme d'un lot comprenant le poussoir de sécurité 22, l'organe rapporté 26 et le capuchon de sécurité 28, ces différents éléments du lot pouvant être montés sur la seringue 12, le cas échéant pré-remplie, comprenant le corps 14 et le piston 18.

L'ensemble de sécurité 13 selon l'invention est en effet très simple à monter sur la seringue 12.

Initialement, on relie le poussoir 22 au piston 18 pour obtenir l'ensemble représenté sur la figure 3 dans lequel le capuchon de protection 24 est emmanché sur l'extrémité d'injection 14D du corps de la seringue.

Le montage de l'ensemble de sécurité 13 sur la seringue 12 est poursuivi en emboîtant l'organe rapporté 26 sur la collerette 20 du corps 14 de la seringue. Cet emboîtement est réalisé par déplacement relatif de l'organe 26 et du corps 14 de la seringue sensiblement perpendiculairement à l'axe de la seringue 12 comme l'autorisent les ouvertures en "U" 38, 42 ménagées dans les flasques proximal 36P et distal 36D de l'organe rapporté.

Dans la configuration de la figure 4, l'organe rapporté 26 est immobilisé axialement par rapport au corps 14 de la seringue mais n'est pas retenu radialement sur ce corps 14. On notera que l'organe rapporté 26 peut être monté sur la collerette 20 en présence du poussoir 22 de cette seringue 12.

L'organe rapporté 26 étant accroché sur le corps 14 de la seringue comme cela est représenté sur la figure 4, on encliquète ensuite le capuchon de sécurité 28 sur l'organe rapporté 26 de manière que ce capuchon 28 recouvre le corps 14 de la seringue et le capuchon 24 de protection de l'aiguille 16, comme cela est représenté sur la figure 1.

Après encliquetage du capuchon de sécurité 28 sur l'organe rapporté 26, ce dernier est immobilisé radialement par rapport au corps 14 de la seringue.

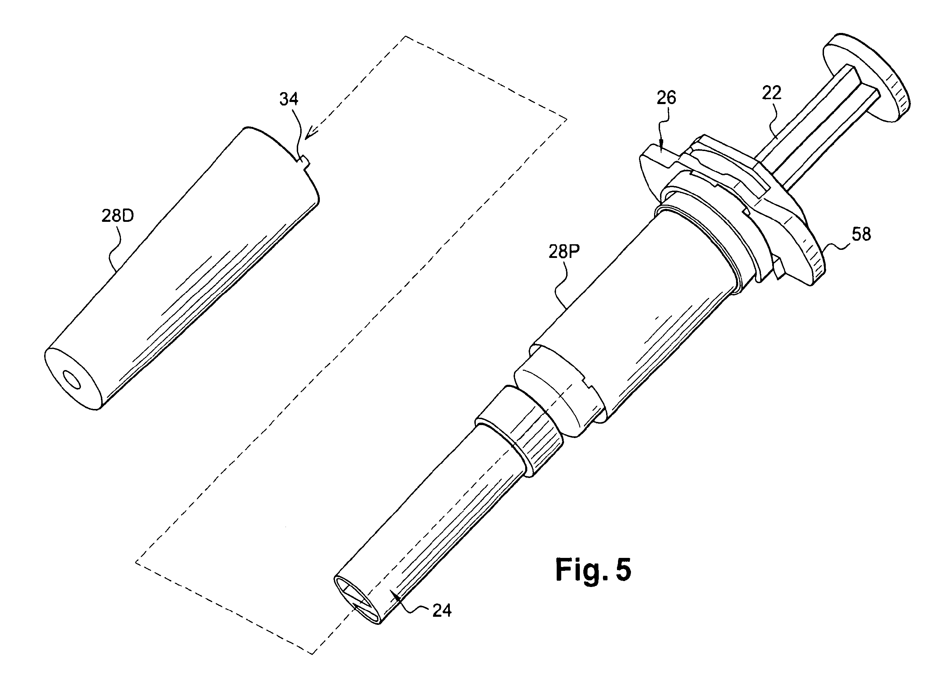

Pour accéder à l'aiguille 16 de la seringue, un utilisateur agit sur l'extrémité de dégagement 28D du capuchon de sécurité de façon à rompre les ponts frangibles 34 et séparer cette partie de dégagement 28D d'avec la partie d'accrochage 28P qui demeure accrochée à l'organe rapporté 26, comme cela est représenté sur la figure 5.

Après retrait de la partie de dégagement 28D du capuchon de sécurité, l'utilisateur peut accéder au capuchon de protection 24 et le retirer normalement de la seringue pour pouvoir utiliser cette seringue dans les conditions habituelles.

L'accès à l'aiguille 16 de la seringue ne pouvant se faire qu'après rupture des ponts frangibles 34 et séparation de la partie de dégagement 28D du capuchon de sécurité d'avec la partie d'accrochage 28P de ce capuchon, un accès malveillant à l'aiguille 16 est facile à détecter.

Par ailleurs, l'organe rapporté 26, formant collet, empêche tout retrait intempestif ou malveillant du piston 18 du corps 14 de la seringue.

Le cas échéant, afin de permettre la détection d'un remplacement frauduleux d'un élément de l'ensemble de sécurité 13, au moins un élément de l'ensemble de sécurité 13, par exemple le capuchon de sécurité 28 ou l'organe rapporté 26, peut comprendre un élément chimique ou un marquage invisible à l'oeil nu mais susceptible d'être détecté à l'aide de moyens adaptés tel qu'un spectromètre à rayonnement ultraviolet ou infrarouge ou encore tel qu'un éclairage par un rayonnement adapté notamment un rayonnement ultraviolet.

Eventuellement, une zone frangible peut être prévue sur le poussoir 22 et/ou l'organe rapporté 26 afin de provoquer la rupture de l'un ou l'autre de ces organes lors d'une tentative de retrait par la force du piston 18 du corps 14 de la seringue.

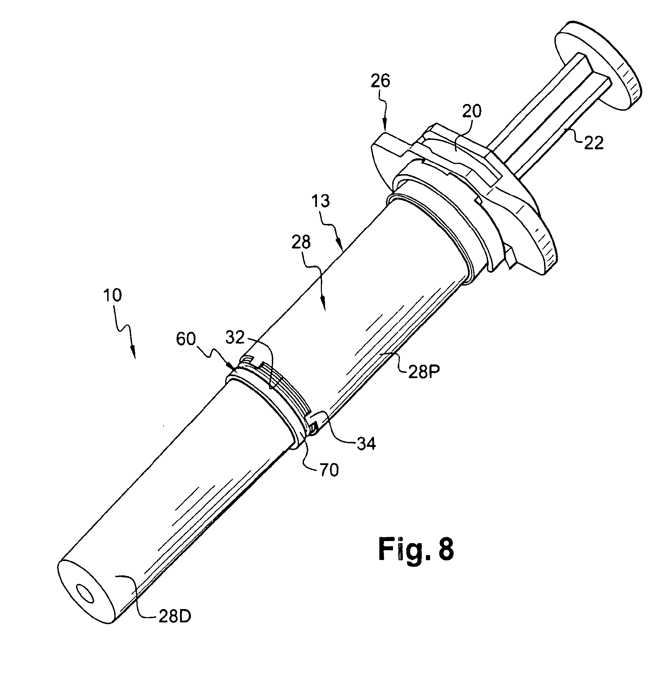

On a représenté sur les figures 8 à 10 un ensemble de seringue selon un second mode de réalisation de l'invention. Sur ces figures, les éléments analogues à ceux des figures 1 à 7 sont désignés par des références identiques.

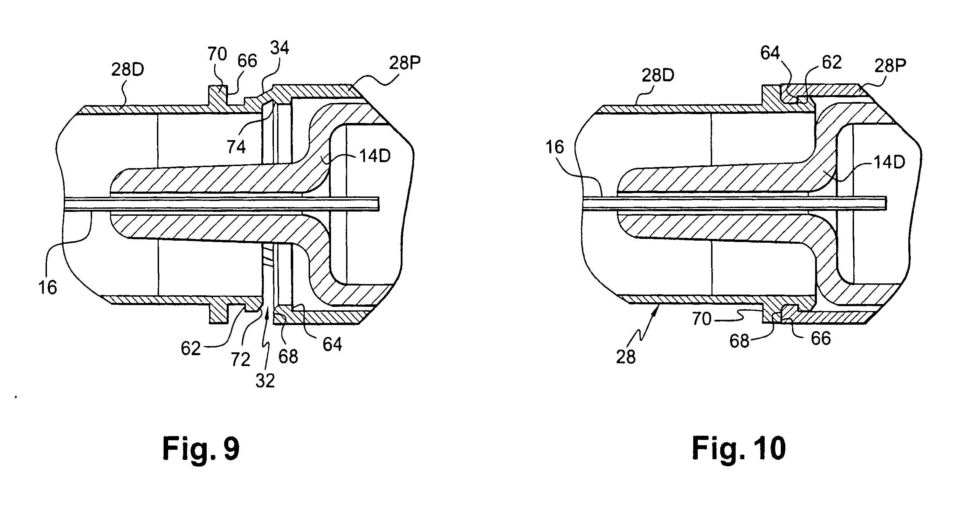

Pour des raisons de clarté, le capuchon de protection 24 n'est pas représenté sur les figures 9 et 10.

Dans ce cas, les parties de dégagement 28D et d'accrochage 28P du capuchon de sécurité 28 sont munies de moyens d'accrochage complémentaires susceptibles de coopérer entre eux après rupture des moyens frangibles 30 de façon à pouvoir, après la première utilisation de la seringue dans les conditions habituelles, isoler de l'extérieur l'extrémité d'injection 14D et l'aiguille 16 de la seringue.

De préférence, comme on peut le voir notamment sur les figures 9 et 10, les moyens d'accrochage complémentaires comprennent des moyens d'encliquetage complémentaires emboîtables 60 munis d'épaulements complémentaires de verrouillage 62, 64 et de butées complémentaires de fin de course d'emboîtement 66, 68 ménagés sur les parties, respectivement, de dégagement 28D et d'accrochage 28P du capuchon de sécurité 28.

Les butées complémentaires de fin de course 66, 68 sont délimitées, respectivement, par une saillie externe 70, par exemple annulaire, ménagée sur l'une des parties du capuchon de sécurité 28, à savoir la partie de dégagement 28D dans l'exemple illustré, et un bord de l'autre des parties de ce capuchon 28, à savoir la partie d'accrochage 28P dans l'exemple illustré, délimitant la fente annulaire 32 avant rupture des ponts frangibles 34 (voir figure 9).

On notera qu'avant leur rupture, les ponts frangibles 34 s'étendent sensiblement entre des rampes d'emboîtement 72, 74 des moyens d'encliquetage complémentaires 60.

Comme dans le premier mode de réalisation de l'invention, pour accéder à l'aiguille 16 de la seringue, un utilisateur agit sur l'extrémité de dégagement 28D du capuchon de sécurité de façon à rompre les ponts frangibles 34 et séparer cette partie de dégagement 28D d'avec la partie d'accrochage 28P qui demeure accrochée à l'organe rapporté 26.

Après retrait de la partie de dégagement 28D du capuchon de sécurité, l'utilisateur peut utiliser la seringue dans les conditions habituelles.

Après cette utilisation, l'utilisateur isole de l'extérieur l'extrémité d'injection 14D et l'aiguille 16 en encliquetant la partie de dégagement 28D sur la partie d'accrochage 28P du capuchon de sécurité, comme cela est représenté sur la figure 10. De préférence, les moyens d'encliquetage complémentaires 60 sont emboîtables les uns dans les autres de façon irréversible.

L'invention ne se limite pas aux modes de réalisation décrits ci-dessus.

En particulier, l'organe rapporté 26, destiné notamment à la retenue du piston 18 dans le corps de la seringue peut avoir une structure différente de celle décrite ci-dessus.

Par ailleurs, le positionnement radial de l'organe rapporté par rapport au corps de la seringue peut être réalisé par des moyens portés par le capuchon de sécurité plutôt que par le secteur de centrage porté par l'organe rapporté.

## Revendications

1. Ensemble de sécurité destiné à équiper une seringue (12) comportant un corps (14) muni d'une extrémité de raccordement à une aiguille d'injection (16), dite extrémité d'injection (14D), et d'une extrémité (14P) d'introduction d'un piston (18), l'ensemble de sécurité (13) étant du type comprenant :
- un organe (26) destiné à être rapporté sur le corps (14) de la seringue (12) de façon solidaire axialement de ce corps (14), et
- un capuchon de sécurité (28), destiné à recouvrir l'extrémité d'injection (14D) du corps (14), muni d'une partie proximale (28P) d'accrochage sur l'organe rapporté (26) et d'une partie distale (28D) de dégagement de l'extrémité d'injection (14D) reliée à la partie d'accrochage (28P) par des moyens frangibles (30),
**caractérisé en ce que** l'organe rapporté (26) forme un organe destiné à retenir le piston (18) à l'intérieur du corps (14).

2. Ensemble de sécurité selon la revendication 1, **caractérisé en ce que** la partie d'accrochage (28P) du capuchon de sécurité (28) forme des moyens de retenue radiale de l'organe rapporté (26) sur le corps (14) de la seringue (12).

3. Ensemble de sécurité selon la revendication 1 ou 2, **caractérisé en ce que** l'organe rapporté (26) comprend au moins un secteur de jupe annulaire, dit secteur de centrage (46), destiné à coopérer avec une surface complémentaire délimitant le corps (14) de la seringue (12) pour positionner radialement l'organe rapporté (26) par rapport au corps (14) de la seringue (12).

4. Ensemble de sécurité selon l'une quelconque des revendications 1 à 3, **caractérisé en ce que** le capuchon de sécurité (28) est accroché sur l'organe rapporté (26) à l'aide de moyens d'encliquetage complémentaires (48) portés par la partie d'accrochage (28P) du capuchon de sécurité (28) et l'organe rapporté (26), ces moyens d'encliquetage complémentaires (48) étant emboîtables l'un dans l'autre de façon irréversible.

5. Ensemble de sécurité selon la revendication 4, **caractérisé en ce que** les moyens d'encliquetage complémentaires (48) comprennent des épaulements complémentaires de verrouillage (50, 52) ménagés, respectivement, sur une extrémité sensiblement annulaire de la partie d'accrochage (28P) du capuchon de sécurité (28) et sur au moins un secteur (54) de jupe annulaire solidaire de l'organe rapporté (26), dit secteur d'encliquetage (54).

6. Ensemble de sécurité selon les revendications 3 et 5 prises ensemble, **caractérisé en ce que** le secteur de centrage (46) est radialement interne par rapport au secteur d'encliquetage (48), l'extrémité sensiblement annulaire de la partie d'accrochage (28P) du capuchon de sécurité (28) étant intercalée radialement entre ces secteurs de centrage (46) et d'encliquetage (48).

7. Ensemble de sécurité selon la revendication 5 ou 6, **caractérisé en ce que** l'épaulement de verrouillage (52) ménagé sur le secteur d'encliquetage (48) est adjacent à des fentes circonférentielles (56) ménagées dans ce secteur d'encliquetage (48).

8. Ensemble de sécurité selon l'une quelconque des revendications précédentes destiné à équiper une seringue (12) comportant un corps (14) muni d'une extrémité (14P) d'introduction d'un piston (18) délimitée par une collerette (20), **caractérisé en ce que** l'organe rapporté (26) comprend des flasques proximal (36P) et distal (36D) destinés à pincer axialement la collerette (20) pour solidariser axialement l'organe rapporté (26) au corps (14) de la seringue (12), le flasque proximal (36P) formant un collet destiné à retenir le piston (18) à l'intérieur du corps (14).

9. Ensemble de sécurité selon les revendications 3 et 8 prises ensemble, **caractérisé en ce que** le flasque distal (36D) porte le secteur de centrage (46).

10. Ensemble de sécurité selon les revendications 4 et 9 prises ensemble, **caractérisé en ce que** le flasque distal (36D) porte les moyens d'encliquetage (52) destinés à coopérer avec les moyens d'encliquetage complémentaires (50) portés par la partie d'accrochage (28P) du capuchon de sécurité (28).

11. Ensemble de sécurité selon l'une quelconque des revendications 8 à 10, **caractérisé en ce que** le flasque distal (36D) comporte une ouverture (42) destinée à l'emboîtement autour du corps (14) de la seringue (12), cette ouverture d'emboîtement (42) débouchant dans une direction sensiblement parallèle aux flasques (36P, 36D), le contour du flasque distal (36D) délimitant cette ouverture d'emboîtement (42) ayant de préférence une forme en "U".

12. Ensemble de sécurité selon l'une quelconque des revendications 8 à 11 destiné à équiper une seringue (12) comportant un corps (14) muni d'un piston (18), **caractérisé en ce qu'**il comprend une tige formant un poussoir (22) destinée à être relié au piston (18) et **en ce que** le flasque proximal (36P) comporte une ouverture (38) destinée au débattement axial du poussoir (22), cette ouverture de débattement (38) débouchant de préférence dans une direction sensiblement parallèle aux flasques (36P, 36D), le contour du flasque proximal (36P) délimitant l'ouverture de débattement (38) formant le collet de retenue du piston (18) et ayant de préférence une forme en "U".

13. Ensemble de sécurité selon la revendication 12, **caractérisé en ce que** le poussoir (22) comporte une extrémité distale (22D) interne au corps (14) munie d'un disque (40) formant une protection anti-perçage du piston (18) et/ou une butée destinée à coopérer avec le flasque proximal (36P) formant collet pour retenir le piston (18) et l'extrémité distale (22D) du poussoir à l'intérieur du corps (14).

14. Ensemble de sécurité selon l'une quelconque des revendications 8 à 13, **caractérisé en ce que** l'organe rapporté (26) comprend des moyens d'immobilisation en rotation (44) destinés à coopérer avec des moyens complémentaires (20R) de la collerette (20) du corps (14) de la seringue (12), ces moyens d'immobilisation en rotation étant munis de préférence de méplats (44) ménagés dans l'organe rapporté (26) de façon à s'étendre axialement entre les deux flasques (36P, 36D).

15. Ensemble de sécurité selon l'une quelconque des revendications précédentes, **caractérisé en ce que** l'organe rapporté (26) est muni de moyens d'appui de doigts d'un utilisateur comprenant de préférence deux ailes de préhension radiales (58) sensiblement diamétralement opposées.

16. Ensemble de sécurité selon l'une quelconque des revendications précédentes, **caractérisé en ce que** le capuchon de sécurité (28) a une forme générale de révolution, les moyens frangibles (30) comprenant une fente annulaire (32), séparant les deux parties (28P, 28D) du capuchon de sécurité (28), cette fente (32) étant interrompue par des ponts frangibles (34) reliant les deux parties (28P, 28D) du capuchon de sécurité (28).

17. Ensemble de sécurité selon l'une quelconque des revendications précédentes, **caractérisé en ce que** les parties de dégagement (28D) et d'accrochage (28P) du capuchon de sécurité (28) sont munies de moyens d'accrochage complémentaires (60) susceptibles de coopérer entre eux après rupture des moyens frangibles (30).

18. Ensemble de sécurité selon la revendication 17, **caractérisé en ce que** les moyens d'accrochage complémentaires comprennent des moyens d'encliquetage complémentaires emboîtables (60) munis d'épaulements complémentaires de verrouillage (62, 64) et de butées complémentaires de fin de course d'emboîtement (66, 68) ménagés sur les parties, respectivement, de dégagement (28D) et d'accrochage (28P) du capuchon de sécurité (28).

19. Ensemble de sécurité selon les revendications 16 et 18 prises ensemble, **caractérisé en ce que** les butées complémentaires de fin de course (66, 68) sont délimitées, respectivement, par une saillie externe (70), par exemple annulaire, ménagée sur l'une (28D) des parties du capuchon de sécurité (28) et un bord de l'autre (28P) des parties du capuchon de sécurité (28) délimitant la fente annulaire (32).

20. Ensemble de sécurité selon l'une quelconque des revendications précédentes, **caractérisé en ce que** le capuchon de sécurité (28) est fabriqué dans un matériau transparent afin notamment de permettre un accès visuel à une étiquette portée par la seringue (12).

21. Ensemble de sécurité selon l'une quelconque des revendications 1 à 20, **caractérisé en ce que** la partie de dégagement (28D) du capuchon de sécurité (28) est destinée à former un capuchon de protection de l'aiguille d'injection (16).

22. Ensemble de sécurité selon l'une quelconque des revendications 1 à 20, **caractérisé en ce que** la partie de dégagement (28D) du capuchon de sécurité (28) est destinée à être reliée à un capuchon de protection de l'aiguille d'injection (16).

23. Ensemble de seringue (12) du type comprenant une seringue (12) comportant un corps (14) muni d'une extrémité de raccordement à une aiguille d'injection, dite extrémité d'injection (14D), et d'une extrémité (14P) d'introduction d'un piston (18), **caractérisé en ce qu'**il comprend de plus un ensemble de sécurité (13) selon l'une quelconque des revendications précédentes équipant la seringue (12).

## Claims

1. A security assembly for fitting to a syringe (12) comprising a body (14) provided with one end for connection to an injection needle (16), referred to as the injection end (14D), and one end (14P) for inserting a piston (18), the security assembly (13) being of the type comprising:
· a member (26) for fitting to the body (14) of the syringe (12) in so as to be constrained axially relative to the body (14); and
· a security cap (28) for covering the injection end (14D) of the body (14), the cap being provided with a proximal portion (28P) for connection to the fitted member (26) and a distal portion (28D) for releasing the injection end (14D) and connected to the connection portion by frangible means (30);
**characterized in that** the fitted member (26) forms a member that is designed to retain the piston (18) inside the body (14).

2. A security assembly according to claim 1, wherein the connection portion (28P) of the security cap (28) forms means for retaining the fitted member (26) radially on the body (14) of the syringe (12).

3. A security assembly according to claim 1 or 2, wherein the fitted member (26) comprises at least one annular skirt sector referred to as a centering sector (46) for co-operating with a complementary surface delimiting the body (14) of the syringe (12) for positioning the fitted member (26) radially relative to the body (14) of the syringe (12).

4. A security assembly according to any one of claims 1 to 3, wherein the security cap (28) is connected to the fitted member (26) by complementary snap-fastening means (48) carried by the connection portion (28P) of the security cap (28) and by the fitted member (26), the complementary snap-fastening means (48) being engageable one in the other in irreversible manner.

5. A security assembly according to claim 4, wherein the complementary snap-fastening means (48) comprise complementary locking shoulders (50, 52) formed respectively on a substantially annular end of the connection portion (28P) of the security cap (28), and on at least one annular skirt sector (54) secured to the fitted member (26), and referred to as the snap-fastening sector (54).

6. A security assembly according to a combination of claims 3 and 5, wherein the centering sector (46) is radially inside the snap-fastening sector (48), the substantially annular end of the connection portion (28P) of the security cap (28) being radially interposed between the centering sector (46) and the snap-fastening sector (48).

7. A security assembly according to claim 5 or 6, wherein the locking shoulder (52) formed on the snap-fastening sector (48) is adjacent to circumferential slots (56) formed in the snap-fastening sector (48).

8. A security assembly according to any one of the preceding claims, for fitting to a syringe (12) comprising a body (14) provided with one end (14P) for inserting a piston (18), said end being defined by a collar (20), wherein the fitted member (26) comprises proximal and distal plates (36P, 36D) for gripping axially on either side of the collar (20) to prevent the fitted member (26) from moving axially relative to the body (14) of the syringe (12), the proximal plate (36P) forming a flange for retaining the piston (18) inside the body (14).

9. A security assembly according to a combination of claims 3 and 8, wherein the distal plate (36D) carries the centering sector (46).

10. A security assembly according to a combination of claims 4 and 9, wherein the distal plate (36D) carries the snap-fastening means (52) for co-operating with the complementary snap-fastening means (50) carried by the connection portion (28P) of the security cap (28).

11. A security assembly according to any one of claims 8 to 10, wherein the distal plate (36D) includes an engagement opening (42) for engaging around the body (14) of the syringe (12), said engagement opening (42) opening out in a direction that is substantially parallel to the plates (36P, 36D), the outline of the distal plate (36D) that defines said engagement opening (42) preferably being U-shaped.

12. A security assembly according to any one of claims 8 to 11, for fitting to a syringe (12) comprising a body (14) provided with a piston (18), the assembly comprising a plunger-forming rod (22) designed to be connected to the piston (18), and wherein the proximal plate (36P) includes a clearance opening (38) for allowing the plunger (22) to move axially, said clearance opening (38) preferably opening out in a direction that is substantially parallel to the plates (36P, 36D), the outline of the proximal plate (36P) that delimits the clearance opening (38) forming the flange for retaining the piston (18) and preferably being U-shaped.

13. A security assembly according to claim 12, wherein the plunger (22) has a distal end (22D) inside the body (14) provided with a disk (40) forming protection against the piston (18) being pierced and/or an abutment for co-operating with the proximal flange-forming plate (36P) to retain the piston (18) and the distal end (22D) of the plunger (22) inside the body (14).

14. A security assembly according to any one of claims 8 to 13, wherein the fitted member (26) includes means (44) for preventing turning movements, which means are designed to co-operate with complementary means (20R) of the collar (20) of the body (14) of the syringe (12), said means for preventing turning movements being preferably provided with flats (44) formed in the fitted member (26) so as to extend axially between the two plates (36P, 36D).

15. A security assembly according to any one of the preceding claims, wherein the fitted member (26) is provided with means against which the fingers of a user can bear, said means preferably comprising two radial finger tabs (58) that are substantially diametrically opposite.

16. A security assembly according to any one of the preceding claims, wherein the security cap (28) is generally in the form of a body of revolution, the frangible means (30) comprising an annular slot (32) between the two portions (28P, 28D) of the security cap, said slot (32) being interrupted by frangible bridges (34) connecting together the two portions (28P, 28D) of the security cap (28).

17. A security assembly according to any one of the preceding claims, wherein the release portion and the connection portion (28P) of the security cap (28) are provided with complementary connection means (60) suitable for co-operating mutually after the frangible means (30) have been ruptured.

18. A security assembly according to claim 17, wherein the complementary connection means comprise mutually engageable complementary snap-fastening means (60) provided with complementary locking shoulders (62, 64) and with complementary end of engagement stroke abutments (66, 68) formed respectively on the release portion (28D) and on the connection portion (28P) of the security cap (28).

19. A security assembly according to a combination of claims 16 and 18, wherein the complementary end of stroke abutments (66,68) are delimited respectively by an outward projection (70), e.g. an annular projection, formed on one of the portions (28D) of the security cap (28), and by an edge of the other portion (28P) of the security cap delimiting the annular slot (32).

20. A security assembly according to any one of the preceding claims, wherein the security cap (28) is made of a transparent material, in particular to give visual access to a label carried by the syringe (12).

21. A security assembly according to any one of claims 1 to 20, wherein the release portion (28D) of the security cap (28) is designed to form a protective cap for the injection needle (16).

22. A security assembly according to any one of claims 1 to 20, wherein the release portion (28D) of the security cap (28) is designed to be connected to a protective cap for the injection needle (16).

23. A syringe assembly (12) of the type comprising a syringe having a body (14) provided with one end (14D) for connection to an injection needle, referred to as the injection end, and one end (14P) for inserting a piston, the assembly further comprising a security assembly (13) according to any one of the preceding claims and fitted to the syringe (12).

## Patentansprüche

1. Sicherheitsanordnung zum Ausrüsten einer Spritze (12), die einen Körper (14) aufweist, der ausgestattet ist mit einem Ende zur Verbindung mit einer Injektionsnadel (16), genannt Injektionsende (14D), und mit einem Ende (14P) zur Einführung eines Kolbens (18), wobei die Sicherheitsanordnung (13) umfasst:
- ein Element (26), das dazu gedacht ist, auf dem Körper (14) der Spritze (12) fest in axialer Richtung dieses Körpers (14) angefügt zu werden, und
- eine Sicherheitskappe (28), zum Umhüllen des Injektionsendes (14D) des Körpers (14), die ausgestattet ist mit einem proximalen Teil (28P) zur Befestigung auf dem Fügeelement (26) und mit einem distalen Teil (28D) zur Freigabe des Injektionsendes (14D), das mit dem Befestigungsteil (28P) durch brechbare Mittel (30) verbunden ist,
**dadurch gekennzeichnet, dass** das Fügeelement (26) ein Element bildet, um den Kolben (18) im Innern des Körpers (14) zu halten.

2. Sicherheitsanordnung nach Anspruch 1, **dadurch gekennzeichnet, dass** das Befestigungsteil (28P) der Sicherheitskappe (28) Mittel zur radialen Halterung des Fügeelements (26) auf dem Körper (14) der Spritze (12) bildet.

3. Sicherheitsanordnung nach Anspruch 1 oder 2, **dadurch gekennzeichnet, dass** das Fügeelement (26) zumindest einen Ringformmantelsektor, genannt Zentriersektor (46), umfasst, der dazu bestimmt ist, mit einer komplementären Oberfläche, die den Körper (14) der Spritze (12) begrenzt, zusammenzuwirken, um das Fügeelement (26) in Bezug auf den Körper (14) der Spritze (12) radial zu positionieren.

4. Sicherheitsanordnung nach einem der Ansprüche 1 bis 3, **dadurch gekennzeichnet, dass** die Sicherheitskappe (28) auf dem Fügeelement (26) mit Hilfe komplementärer Rastmittel (48) befestigt ist, die von dem Befestigungsteil (28P) der Sicherheitskappe (28) und dem Fügeelement (26) getragen werden, wobei diese komplementären Rastmittel (48) in irreversibler Weise ineinandersteckbar sind.

5. Sicherheitsanordnung nach Anspruch 4, **dadurch gekennzeichnet, dass** die komplementären Rastmittel (48) komplementäre Sperrvorsprünge (50, 52) umfassen, die jeweils auf einem im Wesentlichen ringförmigen Ende des Befestigungsteils (28P) der Sicherheitskappe (28) und auf zumindest einem mit dem Fügeelement (26) fest verbundenen Ringformmantelsektor (54), genannt Rastsektor (54), angeordnet sind.

6. Sicherheitsanordnung nach den Ansprüchen 3 und 5 zusammengenommen, **dadurch gekennzeichnet, dass** der Zentriersektor (46) radial innen liegt in Bezug auf den Rastsektor (48), wobei das im Wesentlichen ringförmige Ende des Befestigungsteils (28P) der Sicherheitskappe (28) radial zwischen diesem Zentrier- (46) und diesem Rastsektor (48) eingefügt ist.

7. Sicherheitsanordnung nach Anspruch 5 oder 6, **dadurch gekennzeichnet, dass** der auf dem Rastsektor (48) angeordnete Sperrvorsprung (52) neben umlaufenden Spalten (56) liegt, die in diesem Rastsektor (48) angeordnet sind.

8. Sicherheitsanordnung nach einem der vorstehenden Ansprüche, zum Ausrüsten einer Spritze (12), die einen Körper (14) aufweist, der mit einem Ende (14P) zur Einführung eines von einem Kragen (20) begrenzten Kolbens (18) ausgestattet ist, **dadurch gekennzeichnet, dass** das Fügeelement (26) einen proximalen (36P) und einen distalen (36D) Flansch umfasst, die dazu gedacht sind, den Kragen (20) axial festzuklemmen, um das Fügeelement (26) mit dem Körper (14) der Spritze (12) in axialer Richtung fest zu verbinden, wobei der proximale Flansch (36P) einen Wulst bildet, um den Kolben (18) im Inneren des Körpers (14) zu halten.

9. Sicherheitsanordnung nach den Ansprüchen 3 und 8 zusammengenommen, **dadurch gekennzeichnet, dass** der distale Flansch (36D) den Zentriersektor (46) trägt.

10. Sicherheitsanordnung nach den Ansprüchen 4 und 9 zusammengenommen, **dadurch gekennzeichnet, dass** der distale Flansch (36D) die Rastmittel (52) trägt, die zum Zusammenwirken mit den komplementären Rastmitteln (50) bestimmt sind, die vom Befestigungsteil (28P) der Sicherheitskappe (28) getragen werden.

11. Sicherheitsanordnung nach einem der Ansprüche 8 bis 10, **dadurch gekennzeichnet, dass** der distale Flansch (36D) eine Öffnung (42) aufweist, die zum Fügen um den Körper (14) der Spritze (12) bestimmt ist, wobei diese Fügeöffnung (42) in eine Richtung mündet, die im Wesentlichen parallel zu den Flanschen (36P, 36D) ist, wobei die Kontur des distalen Flansches (36D) diese Fügeöffnung (42) begrenzt, die vorzugsweise U-förmig ist.

12. Sicherheitsanordnung nach einem der Ansprüche 8 bis 11, zum Ausrüsten einer Spritze (12), die einen Körper (14) aufweist, der mit einem Kolben (18) ausgestattet ist, **dadurch gekennzeichnet, dass** er einen ein Schiebeteil (22) bildenden Stiel umfasst, der dazu bestimmt ist, mit dem Kolben (18) verbunden zu werden, und dass der proximale Flansch (36P) eine Öffnung (38) aufweist, die für den axialen Weg des Schiebeteils (22) gedacht ist, wobei diese Wegöffnung (38) vorzugsweise in eine Richtung mündet, die im Wesentlichen parallel zu den Flanschen (36P, 36D) ist, wobei die Kontur des die Wegöffnung (38) begrenzenden proximalen Flansches (36P) den Rückhaltewulst für den Kolben (18) bildet und vorzugsweise U-förmig ist.

13. Sicherheitsanordnung nach Anspruch 12, **dadurch gekennzeichnet, dass** das Schiebeteil (22) ein innerhalb des Körpers (14) befindliches distales Ende (22D) aufweist, das mit einer Scheibe (40) versehen ist, die einen Durchstoß-Schutz für den Kolben (18) und/oder einen Anschlag bildet, der dazu bestimmt ist, mit dem einen Wulst bildenden proximalen Flansch (36P) zusammenzuwirken, um den Kolben (18) und das distale Ende (22D) des Schiebeteils im Inneren des Körpers (14) zu halten.

14. Sicherheitsanordnung nach einem der Ansprüche 8 bis 13, **dadurch gekennzeichnet, dass** das Fügeelement (26) Mittel (44) zur Verdrehsicherung umfasst, die dazu bestimmt sind, mit komplementären Mitteln (20R) des Kragens (20) des Körpers (14) der Spritze (12) zusammenzuwirken, wobei diese Mittel zur Verdrehsicherung vorzugsweise mit Flachstellen (44) versehen sind, die so im Fügeelement (26) angeordnet sind, dass sie sich axial zwischen den beiden Flanschen (36P, 36D) erstrecken.

15. Sicherheitsanordnung nach einem der vorstehenden Ansprüche, **dadurch gekennzeichnet, dass** das Fügeelement (26) mit Mitteln zum Stützen von Fingern eines Benutzers ausgestattet ist, die vorzugsweise zwei radiale Griffflügel (58) umfassen, die sich im Wesentlichen diametral gegenüberliegen.

16. Sicherheitsanordnung nach einem der vorstehenden Ansprüche, **dadurch gekennzeichnet, dass** die Sicherheitskappe (28) eine allgemeine Drehform besitzt, wobei die brechbaren Mittel (30) einen ringförmigen Spalt (32) umfassen, der die beiden Teile (28P, 28D) der Sicherheitskappe (28) trennt, wobei dieser Spalt (32) durch brechbare Brücken (34) unterbrochen ist, welche die beiden Teile (28P, 28D) der Sicherheitskappe (28) verbinden.

17. Sicherheitsanordnung nach einem der vorstehenden Ansprüche, **dadurch gekennzeichnet, dass** die Teile zur Freigabe (28D) und zur Befestigung (28P) der Sicherheitskappe (28) mit komplementären Befestigungsmitteln (60) ausgestattet sind, die sich eignen, um nach dem Bruch der brechbaren Mittel (30) miteinander zusammenzuwirken.

18. Sicherheitsanordnung nach Anspruch 17, **dadurch gekennzeichnet, dass** die komplementären Befestigungsmittel einpassbare komplementäre Rastmittel (60) umfassen, die mit komplementären Sperrvorsprüngen (62, 64) und mit komplementären Endeinpassungsanschlägen (66, 68) ausgestattet sind, die auf den Teilen jeweils zur Freigabe (28D) und zur Befestigung (28P) der Sicherheitskappe (28) angeordnet sind.

19. Sicherheitsanordnung nach den Ansprüchen 16 und 18 zusammengenommen, **dadurch gekennzeichnet, dass** die komplementären Endanschläge (66, 68) jeweils begrenzt sind durch ein äußeres vorspringendes, beispielsweise ringförmiges, Teil (70), das auf dem einen (28D) der Teile der Sicherheitskappe (28) angeordnet ist, und einen Rand des anderen (28P) der Teile der Sicherheitskappe (28), begrenzend den ringförmigen Spalt (32).

20. Sicherheitsanordnung nach einem der vorstehenden Ansprüche, **dadurch gekennzeichnet, dass** die Sicherheitskappe (28) in einem transparenten Material hergestellt ist, um insbesondere einen visuellen Zugriff auf ein Etikett zu ermöglichen, das von der Spritze (12) getragen wird.

21. Sicherheitsanordnung nach einem der Ansprüche 1 bis 20, **dadurch gekennzeichnet, dass** das Freigabeteil (28D) der Sicherheitskappe (28) dazu gedacht ist, eine Schutzkappe der Injektionsnadel (16) zu bilden.

22. Sicherheitsanordnung nach einem der Ansprüche 1 bis 20, **dadurch gekennzeichnet, dass** das Freigabeteil (28D) der Sicherheitskappe (28) dazu bestimmt ist, mit einer Schutzkappe der Injektionsnadel (16) verbunden zu werden.

23. Spritzenanordnung (12), umfassend eine Spritze (12) mit einem Körper (14), der ausgestattet ist mit einem Ende zur Verbindung mit einer Injektionsnadel, genannt Injektionsende (14D), und mit einem Ende (14P) zur Einführung eines Kolbens (18), **dadurch gekennzeichnet, dass** sie weiterhin eine Sicherheitsanordnung (13) nach einem der vorstehenden Ansprüche umfasst, mit der die Spritze (12) ausgerüstet ist.
